(19) Europäisches Patentamt
European Patent Office
Office européen des brevets

(11) **EP 2 281 528 B1**

(12) **EUROPEAN PATENT SPECIFICATION**

(45) Date of publication and mention
of the grant of the patent:
**06.05.2020  Bulletin 2020/19**

(21) Application number: **09754002.5**

(22) Date of filing: **18.05.2009**

(51) Int Cl.:
***A61C 1/00*** *(2006.01)*      ***A61C 17/00*** *(2006.01)*
***A61K 6/864*** *(2020.01)*

(86) International application number:
**PCT/ES2009/070163**

(87) International publication number:
**WO 2009/144348 (03.12.2009 Gazette 2009/49)**

(54) **SYSTEM FOR LASER TREATMENT OF DENTAL CARIES**

SYSTEM ZUR LASERBEHANDLUNG VON KARIES

SYSTÈME POUR LE TRAITEMENT DE LA CARIE DENTAIRE PAR LASER

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **30.05.2008  ES 200801642 P**

(43) Date of publication of application:
**09.02.2011  Bulletin 2011/06**

(73) Proprietors:
- **José Chafer, José Luis**
  **46220 Picassent (Valencia) (ES)**
- **Torres Celda, Vicente Manuel**
  **46004 Valencia (ES)**

(72) Inventor: **TORRES ZARAGOZÁ, Vicente Manuel**
**E-46004 Valencia (ES)**

(74) Representative: **Ballester Cañizares, Rosalia**
**Ballester Intellectual Property SLP**
**Avda. de la Constitución 16, 1º D**
**03002 Alicante (ES)**

(56) References cited:
**EP-B1- 0 390 951      DE-A1- 4 339 779**
**ES-T3- 2 128 442      IT-A1- RM 920 633**
**US-B1- 6 334 891**

- **GRAHAM J. MOUNT ET AL. LA CONSERVACION AND RESTAURACIÓN OF THE ESTRUCTURA DENTAL 1999, page 102, XP008147696**

EP 2 281 528 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD OF THE INVENTION**

The invention belongs to the field of dentistry, specifically, the clinical treatment, both preventive and corrective, of dental cavities.

**STATE OF THE ART**

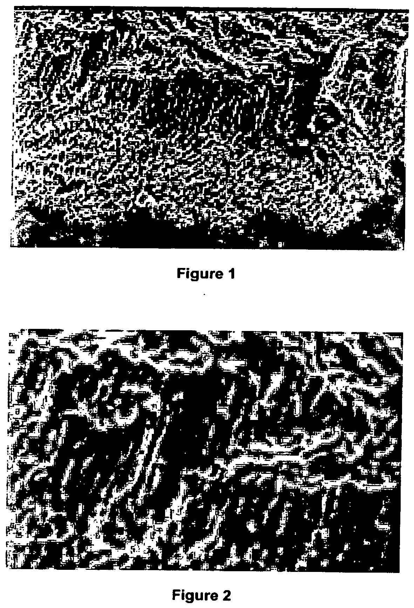

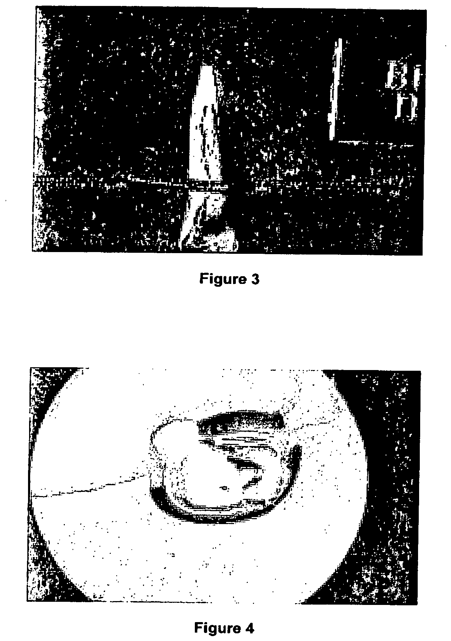

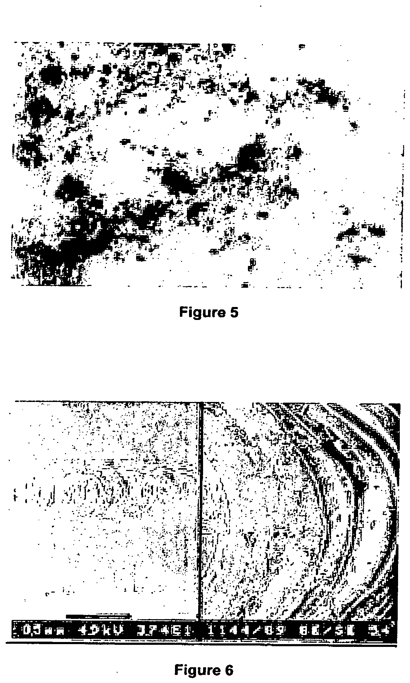

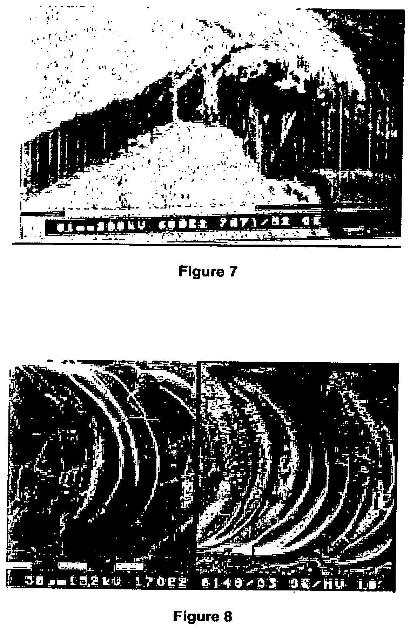

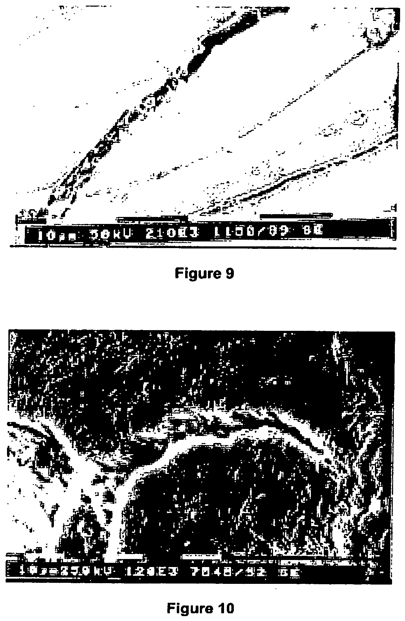

Dental enamel is the hardest, most mineralized human tissue; its mechanical, physical and chemical properties are dependent on and, in turn, encompass, from its mineral composition to its structural order ([1]). Dental enamel plays a very active role in the de-mineralisation and re-mineralisation ([4] and [5]); these dynamic properties are dependent on both the porosity and the electrochemical characteristics thereof ([6] and [7]).
A calcium phosphate apatite, in the form of hydroxyapatite, is the basic component of dental enamel ([2]). Hydroxyapatite crystals are organised in densely condensed prismatic structures, perpendicularly arranged toward the outer surface. Its structural arrangement gives teeth considerable mechanical resistance (Figures 1 and 2).
The small quantities of organic matter of enamel (structural proteins, lipids and carbohydrates) located in interprismatic spaces, may play a significant role in the plasticity of such a rigid structure ([3]). However, enamel is also an organic tissue that is involved in both the transport of ions and solutions from saliva and in the de-mineralization and re-mineralisation process ([4] and [5]).
Such dynamic properties are dependent on both the porosity and the electrochemical characteristics of enamel, that is, of its potential membrane and its fixed charge ([6] and [7]). The effect of laser irradiation on dental enamel, a field that started in the 1970's, has been the subject of several research projects in recent years ([2], [8], [9] and [10]). Some prior dental publications have applied several types of laser, primarily Carbon dioxide ($CO_2$), Neodymium Yag (Nd:YAG), Argon (Ar) or Erbium-Yag ((Er:YAG) lasers, which have been used under different conditions, depending on the desired results ([11] to [17]).
In the case of the Nd:YAG laser, it has been normally applied to soft tissues (surgery) and hardly ever on hard tissues, since, when it has been used in this manner, it has been applied on an enamel that had to be previously coated and painted with colourings agents, in order to increase its absorption energy. These colourings agents cause a deeply unsightly effect due to the residues that remain trapped in the adamantine structure and, moreover, numerous cracks appear in the dental enamel, always after the latter is exposed to and irradiated by the laser ([18], [19] and [20]) (figures 2-10).
These undesirable effects have limited scientific progress and research on lasers with might have the potential for dental usage on hard tissues, especially Nd:YAG lasers. To this, one must add the fact the use of the Nd:YAG laser in dentistry has been limited to the direct application thereof on the tissue to be treated, and it has not been previously used for the irradiation and microfusion of dental cementing and restorative materials.
In this invention, by applying certain modifications and new parameters, it has not been necessary to previously coat or paint the tooth with absorbent colouring agents, and the formation of cracks in the dental enamel following treatment with the Nd:YAG laser has also been prevented.
As regards corrective treatments of dental cavities, the restoration and cementing of human teeth is generally performed with materials (silver amalgams, compound resins, composites, glass ionomers) whose composition, hardness, abrasion resistance, aesthetics, etc., thereof are different from those of the tooth ([21]). As shown by numerous authors, all the materials that are currently used are somewhat toxic for the dental pulp and some of them are toxic for the human body ([22]); hence the controversy regarding the toxicity of the mercury vapours from silver amalgams and the oestrogenic character of resins, fissure, sealants and composites.
Currently, amalgams and composites are applied to teeth by fixation in a mechanical-retentive and adhesive manner, respectively ([23] and [24]). Hydroxyapatite is a mineral that is present in nature and in the industry, whose chemical composition hereof is the same as that of dental enamel and very similar to that of dentin, elements that belong to the same family of apatites, for which reason their composition, hardness, abrasion resistance, aesthetics, etc., are practically identical to those of teeth ([24]).
Moreover, mineral hydroxyapatite has no toxicity whatsoever, which prevents the clinical failures that take place with the current materials ([25]). For the reason described, mineral hydroxyapatite is considered to be the ideal new material for restoration and cementing, since it is the same mineral that forms teeth (both enamel and dentin), although it is true that these crystallise in the enamel in the form of prisms ([27]).
The fixation thereof to the tooth may be performed by microfusion (laser irradiation), that is, by fusing it to the tooth to become a part thereof; this fixation is much superior to the mechanical-retentive and adhesive fixation of amalgams, glass ionomers and composites ([24] and ([26]). This process is the one applied in this invention.
On the basis of the considerations explained above, a new process, preventive as well as corrective, has been developed for the treatment of cavities which is offered as an alternative to current techniques, since it overcomes the limitations posed by the latter. On the one hand, the invention consists of applying a new technology, the Nd:YAG laser, on the dental surface or enamel in order to prevent caries. On the other hand, the laser may also be applied on a biomaterial preferably composed hydroxyapatite, which is used as a new dental restorative and cement material, and is applied on the dental surface to be treated prior to applying the laser. In either case, the laser is irradiated under specific conditions and parameters that confer great advantages thereto as compared to other inventions.

In this regard, it has been demonstrated that European Patent Application No. EP 0392951 A2 discloses a treatment for cavities similar to the one presented herein. However, both methods are significantly different, and this invention represents a clear advance with respect to the achievements of said European Application; moreover, evident technical differences between the processes and materials disclosed in both documents have been observed. The main differences between documents are summarised below:

- The process and the equipment disclosed in document EP 0392951 A2 have been specifically designed for a corrective treatment of dental cavities, unlike this invention, which additionally represents an advance in preventive treatment.
- The results described in document EP 0392951 A2 are not as conclusive as those obtained by means of this invention (occasionally, not even the desired results are obtained).
- The laser beam of the invention disclosed in EP 0392951 A2 does not have a variable focussing and defocusing system, that is, it is always focussed.
- The processed disclosed in document EP 0392951 A2 produces all the undesirable side effects caused by this type of dental treatment:

  • It melts the hydroxyapatite and the dental enamel in the form of superimposed slices.
  • Concave depressions form on the surfaces whereto it is applied,
  • Large cracks form due the abrupt heating and cooling of the biomaterial and the tooth,
  • It produces an unaesthetic appearance of the material treated since impurities and colouring agents are captured

- The process disclosed in document EP 0392951 A2 leads to a greater fragility of the tooth.
- The process of EP 0392951 A2 leads to an easy accumulation of bacterial plaque and filtration with penetration of lactic acid into the cracks produced; for this reason, dental cavities, of a larger size, reproduce and reappear.
- The process disclosed in document EP 0392951 A2 causes a great reflexion of the laser irradiation due to the lack of separation of the mineral surface to be treated, with the consequent need to increase the laser power and more significant side effects.
- The paste disclosed in document EP 0392951 A2 as a corrective treatment for dental cavities is composed of: 80% ceramics (a material that is very different in structure and composition from the material that forms teeth), and only 20% mineral hydroxyapatite, which should be the most important element in the composition of the paste, since teeth are completely made up of this mineral (from the apatite family).

Prior German document DE 43 39 779 A1 discloses a reinforcement agent for the treatment of dental cavities, where the use of Nd:YAG laser for the treatment of said cavities is specifically mentioned, and such reinforcement agent facilitates the application of said laser without any side effects for the tooth. Neither the use of hydroxyapatite nor the use of a variable focussing laser, type Neodymium YAG or equivalent are disclosed.

DESCRIPTION OF THE INVENTION

This invention relates to a Systeme for laser treatment of dental caries. Preferred embodiments are specified in the dependent claims.

Preferably, the dental surface or enamel is subject to an Nd:YAG laser irradiation with a degree of multi-focality and multiple focus which is variable and regulated at will from maximum focusing of the laser beam to complete defocusing.

The variable focusing and defocusing is regulated at will and takes place on the optical fibre wherethrough the Nd:YAG laser is transmitted. The fact that different types of focus or focusing maybe applied at will on the laser beam is useful to eliminate and prevent many of the undesirable side effects that take place when there are abrupt thermal changes (heating and cooling) caused by irradiation on the dental enamel surface (and, as it will be seen further below in a preferred embodiment, also on a biomaterial that is applied to said surface). This undesirable side effects take place when the laser beam is focused, the most significant being the following:

  i. Concave depressions in the form of super imposed slices of fussed and melted enamel on the treated surface, due to the abrupt heating.

*ii.* Cracks of a very variable size on the same surface, due to the abrupt cooling.

*iii.* Capture and incorporation of impurities and colouring agents in the enamel, which gives it an unaesthetic appearance.

The possibility to regulate the focus or focusing of the laser beam at will allows for the treatment, whether preventive or corrective, of dental cavities and each of its applications to make all the undesirable side effects listed to disappear, leading to a microscopic, uniform, smooth, waterproof, very aesthetic microfusion, all of which increases the hardness and microhardness of the enamel (and, as it will be seen further below, also of the biomaterial that may be applied on the enamel).

The dental surface or enamel is subjected to an Nd:YAG laser irradiation which density is between 3 and 30 $J/mm^2$, both limits included, and, more preferably 4 $J/mm^2$. In another preferred embodiment, the laser radiation frequency is between 1 and 10 kHz, both limits included, and, more preferably 1kHz.

The dental surface or enamel is subjected to an Nd:YAG irradiation which pulse energy is between 1 y 10 mJ/pulse, both limits included, and, more preferably, 2 mJ/pulse.

The laser irradiation spot size is between 1 and 6 mm, both limits included, and, more preferably, 3 mm or 5 mm.

The process disclosed is characterised in that the dental surface or enamel is subjected to an Nd:YAG laser irradiation which exposure time is between 1 and 6 seconds, both limits included, and, more preferably, 2 seconds.

The dental surface or enamel is subjected to an Nd:YAG laser irradiation which peak power is between 70 and 125 kW, both limits included, and, more preferably, 120 kW.

The dental surface or enamel is subjected to an Nd:YAG laser irradiation which pulse width is between 100 and 130 ns, both limits included, and, more preferably, 110 ns.

Another condition for the laser irradiation is that the mean energy is between 10 and 50 W, both limits included, and, more preferably, 13 W.

The dental surface or enamel is subjected to a Nd:YAG laser irradiation which total energy per application is between 15 and 220 J, both limits included, and, more preferably, 26 J.

Preferably in the process, prior to the laser irradiation, the dental surface or enamel to be treated is etched with an acid for a time between 0.5 and 2 minutes, primarily in order to reduce the light reflection thereof. Said acids is preferably orthophosphoric acid, and the etching time is 1 minute.

The process disclosed so far is used for the total preventive treatment of dental cavities. If a step is added to the process wherein a biomaterial is applied on the dental surface or enamel or in dentin prior to the Nd:YAG laser irradiation, the process may also be applied as a corrective treatment for dental cavities. Said application of the biomaterial is preferably performed in layers, with a thickness of between 0.5 and 2 mm, both limits included, and, more preferably, with a thickness of 1 mm.

If a biomaterial is applied on the dental surface or enamel to be treated, both (the biomaterial and dental surface) are subjected to an Nd:YAG laser irradiation with a degree of multi-focality and multiple focus, which is variable and regulated at will from maximum focusing of the laser beam to complete defocusing. In this way, the biomaterial is irradiated and focus to the dental enamel surface or the dentin, until complete fixation by microfusion is achieved. As mentioned above in regards to the preventive treatment for dental cavities, the variable focusing and defocusing is regulated at will and takes place on the optical fibre wherethrough the Nd:YAG laser is transmitted. The fact that different types of focus or focusing may be applied on the laser beam is useful to eliminate and prevent the same undesirable side effects that were listed upon describing the characteristics of the preventive treatment. In a preferred embodiment, the laser radiation parameters are: density 28 $J/mm^2$, frequency: 5 kHz. pulse energy: 8 mJ/pulse, spot size: 3 mm, exposure time: 5 ss, peak power 72 kW, pulse width: 120 ns, average energy: 40 W, and total energy per application: 200 J.

Preferably, prior to applying the biomaterial, the decayed tissue is eliminated; and, more preferably, the elimination is performed by means of diamond drills and tungsten carbide on a water-cooled turbine.

Preferably, after eliminating the decayed tissue and prior to applying the biomaterial, the dental area to be treated is weakly etched with an acid, preferably orthophosphoric acid, for a time between 20 seconds and 1 minute, preferably 30 seconds for the dental enamel and 15 seconds for the dentin.

In another preferred examplary process not forming part of the invention the corrective treatment for dental cavities disclosed so far comprises the following steps:

(i) Eliminating the decayed tissue;
(ii) Weakly etching the dental enamel surface or the dentin to be treated with orthophosphoric acid;
(iii) Applying a biomaterial on said surface, and
(iv) Irradiating with a Neodymium-YAG laser,

*a*. density: 28 J/mm2;
*b*. frequency: 5 kHz;

*c*. pulse energy: 8 mJ/pulse;
*d*. spot size: 3 mm;
*e*. exposure time: 5 s;
*f.* peak power: 72 kW;
*g*. pulse width: 120 ns;
*h*. average energy: 40 w, and
*i*. total energy per application: 200 J.

This disclosure also relates to a dental cementing and restorative biomaterial to be used in the corrective treatment described above, characterised in that it is composed of at least mineral hydroxyapatite. Preferably, the content by weight of hydroxyapatite is at least 75%.

In order to facilitate the application thereof the biomaterial preferably consists of a paste that contains at least dense, powdered, micronised mineral hydroxyapatite, mixed with gelatine.

In a particular embodiment, the hydroxyapatite maybe mixed with other substances that favour the application thereof and, moreover, leave no residues upon being irradiated with Nd:YAG laser. One example is the use of gelatines.

## DESCRIPTION OF THE FIGURES

Figure 1. Scanning electron microscope (SEM) view: Normal appearance of human dental enamel. Structural distribution in the form of prisms perpendicular to the outer surface.

Figure 2. SEM: Normal appearance of human dental enamel. The distribution of the hydroxyapatite prisms is perpendicular to the outer enamel surface, giving the tooth considerable mechanical resistance.

Figure 3. Tooth (incisor) Impregnated with colouring agent in order to increase the laser absorption energy. The colouring agent that was applied on this tooth was Indian ink.

Figure 4. Tooth with absorbent colouring agent (Indian ink) prepared on epoxy resin.

Figure 5. Unaesthetic appearance of the enamel after being laser irradiated. Residues of colouring agent trapped in the adamantine structure of the dental enamel.

Figure 6. SEM: Superimposed impacts of enamel fused with laser in the form of slices. Appearance of the cracks produced.

Figure 7. SEM: Appearance of the enamel crack following laser irradiation.

Figure 8. SEM: Appearance of the fused, impacted enamel in slices and formation of cracks following laser irradiation.

Figure 9. SEM: Appearance of the crack formed in the enamel following application of the laser.

Figure 10. SEM: Appearance of the fused enamel and crack produced instantaneously with the application of the laser.

Figure 11. SEM: Appearance of the normal enamel surface after preparing the dental sample.

Figure 12. Tooth (canine) completely covered with acid-resistant wax and with two windows in the enamel, a control window and a window for the application of the Nd:YAG laser.

Figure 13. Dental crown (molar) previously cut to separate it from the root and completely coated with acid-resistant wax and the two windows in the enamel, a control window and a window for the application of the Nd:YAG laser.

Figure 14. SEM: The loss of the enamel surface structural characteristics on the upper area treated with laser (microfused enamel), and the structural preservation in the untreated lower area may be observed. No cracks formed following application of the laser.

Figure 15. SEM: Appearance of the loss of the enamel surface structural characteristics in the upper area, treated with laser (microfused enamel), and structural preservation in the untreated lower area. No cracks formed following laser irradiation.

Figure 16. SEM: Appearance of the enamel microfusion in the lower area, treated with laser. It was not accompanied by the formation of cracks.

Figure 17. SEM: Normal structural distribution of dental enamel, represented by the perikymata, forming small waves.

Figure 18. SEM: Normal structural distribution of dental enamel, represented by the perikymata, forming small waves.

Figure 19. SEM: Normal structural distribution of dental enamel, represented by the perikymata, forming small waves

Figure 20. SEM: Normal structural distribution of dental enamel, represented by the perikymata, forming small waves.

Figure 21. Tooth (molar) in epoxy resin for the roughness measurement.

Figure 22. SEM: Surface microfusion of the enamel prisms produced by the laser and loss of roughness and surface roughness.

Figure 23. SEM: Loss of the surface structure of the enamel prisms due to the microfusion thereof produced by the treatment with Nd:YAG laser. There is a loss of roughness and surface roughness.

Figure 24. Graph of the roughness profiles of dental samples (a), (b) and (c).

Figure 25. SEM: Amorphous, dense, compact structure of mineral hydroxyapatite fused by laser and used as a dental cementing and restorative material.

Figure 26. SEM: The left-side area shows the mineral hydroxyapatite fused by the laser and its junction to the dental enamel of the untreated right-hand area, with the normal surface characteristics thereof. There is a total absence of cracks in the treated area.

Figure 27. SEM: The lower area shows the mineral hydroxyapatite fused by the laser and its junction to the dental enamel in the untreated upper area, with the normal surface characteristics thereof. There is a total absence of cracks in the treated area.

Figure 28. SEM: Mineral hydroxyapatite fused by the laser with its amorphous surface appearance.

Figure 29. SEM: Absence of cracks in the upper area, treated with laser; we may observe the junction by fusion of mineral hydroxyapatite with the normal dental enamel of the lower area.

Figure 30. Zeiss polarised-light optical microscope (Germany), for the study of teeth at lower magnifications.

Figure 31. Optical microscope (O/M): We may observe the microfusion junction area (arrows) of fused mineral hydroxyapatite and the dental structure (dentin and enamel) in two cements (one of them identified with a blue gel).

Figure 32. O/M: We may observe the microfusion junction areas (arrows) of fused mineral hydroxyapatite and the dental structure (dentin and enamel) in a cement. No gelatin residues from the paste are observed.

Figure 33. O/M: We may observe the mircrofusion junction areas (arrows) of fused mineral hydroxyapatite and the dental structure (dentin and enamel) in a cement. No gelatine residues from the paste are observed.

Figure 34. O/M: We may observe the microfusion junction areas (arrows) of fused mineral hydroxyapatite and the dental structure (dentin and enamel) in a cement.

Figure 35. O/M: We may observe the microfusion junction areas (arrows) of fused mineral hydroxyapatite and the dental structure (dentin and enamel) in a cement.

Figure 36. O/M: We may observe the microfusion junction areas (arrows) of fused mineral hydroxyapatite (H) and the dental structure (dentin and enamel) in a restoration.

Figure 37. O/M: We may observe the microfusion junction areas (arrows) of fused mineral hydroxyapatite (H) and the dental structure (dentin and enamel) in a restoration.

## EMBODIMENT EXAMPLES

**EXAMPLE 1. Preventive treatment for dental cavities. Study of the effects of the Neodymium Yag laser upon being applied on the human dental surface or enamel.**

<u>Nature and processing of the sample</u>

In order to perform the process of this invention, a sample was selected composed of 400 healthy human teeth, extracted for orthodontics reasons and carefully selected on the basis of the criterion that they did not present any lesion that might mask the morphological effects of the treatment.

The teeth were fixed with 2.5% of glutraaldehyde in 0.1 M buffered sodium phosphate (ph 7.02) at a temperature of 4°C for 12 hours. Subsequently, they were washed in the same buffered in 3 baths, 10 minutes each, and, later, with distilled water ([28]).

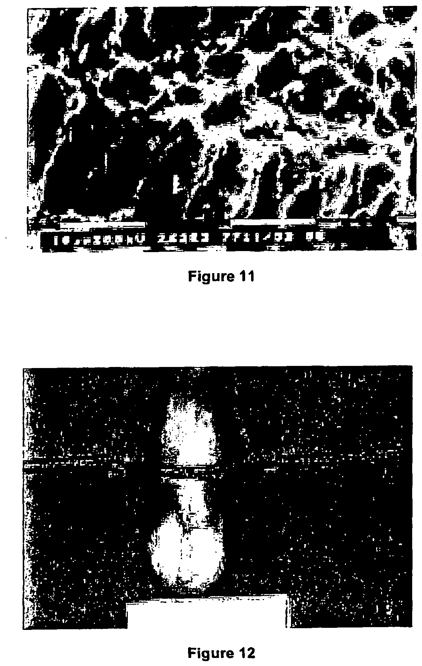

Subsequently, they were washed with 12% Sodium Hypochlorite for 1 hour in order to remove the organic matter from the surface and, finally, all the teeth were weakly etched in a solution of 0.5 M orthophosphoric acid for 1 minute, and later rinsed with abundant distilled water ([28], [29], [30] and [31]) (Figure 11).

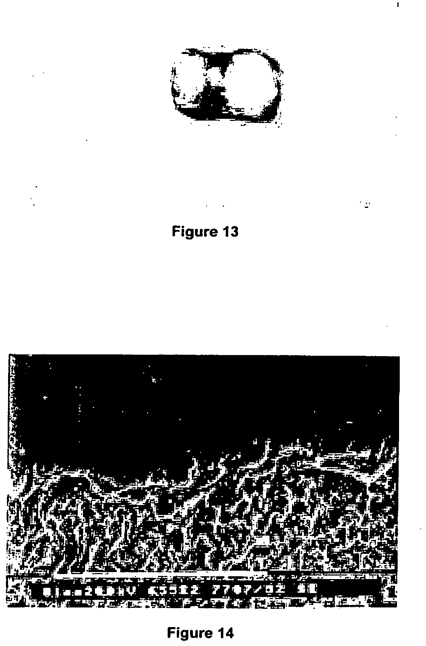

The 400 teeth in the sample were coated with acid-resistant wax, leaving 2 uncoated square windows on the enamel of each tooth: one of the windows was used as a control and the other was irradiated with the Nd:YAG laser (Figures 12 and 13).

After this was done, each of the teeth was individually placed in 50 ml of a de-mineralising solution (pH = 4.5) containing 5% of hydroxyethylcellulose, 0.1 M of lactic acid, 1.5 mM of calcium chloride and 1.5 mM os sodium phosphate, at 37°C for 60 days, in order to form artificial cavity lesions.

<u>Nd:YAG laser irradiation contidions:</u>

The corresponding window of each of the 400 teeth in the sample was subjected to irradiation with a DCR-2 Nd :YAG Laboratory Laser System from Quanta-Ray (United Kingdom).

The following parameters were used for the laser irradiation:

- Density: 4 J / mm$^2$.
- Frequency: 1 kHz.
- Pulse energy: 2 mJ / pulse.
- Spot size: 3 mm.
- Exposure time: 2 s.
- Pick power: 120 kW.
- Pulse width: 110 ns.
- Average energy: 13 w.
- Total energy per application: 26 J.

<u>Post-treatment measurements</u>

*Observations with the scanning electron microscope analyser:*

From the total sample, 300 teeth were randomly processed in accordance with the conventional SEM examination method and coated or metallised with gold in the Bio-Rad metalliser, model SC 5.000 (Holland). This dental sample was examined with a Philips 515 SEM (Holland) at 20 kV, as well as with the Edax chemical elemental analyser from Philips (Holland) for the SEM.

*Hardness measurements:*

The other sample, composed of a 100 teeth, which was not processed to be examined under the SEM, was used. Each of the teeth was embedded in epoxy resin such that a portion of a cross section of the lesion and the normal inner layer of the enamel were exposed. This surface was connected in a Buehler Motopol 8 polishing machine (Germany) using a metallographic paper grid.

Subsequently, they were serially polished with 15 m$\mu$, 6 m$\mu$ and 1 m$\mu$ with a Buehler diamond abrasive (Germany) and, subsequently, with Buelher Metadi diamond spray (Germany) on a Buehler polishing cloth (Germany). A diamond tip under a 10-g load was used in a Matsuzawa MTH-I (Japan). The results for the KDN hardness were calculated using the equation:

$$KHN = 14230 \times F/L2$$

where L is the entry length of each depression in the diamond, in microns, and F is the applied force in grams.

*Results of the treatment:*

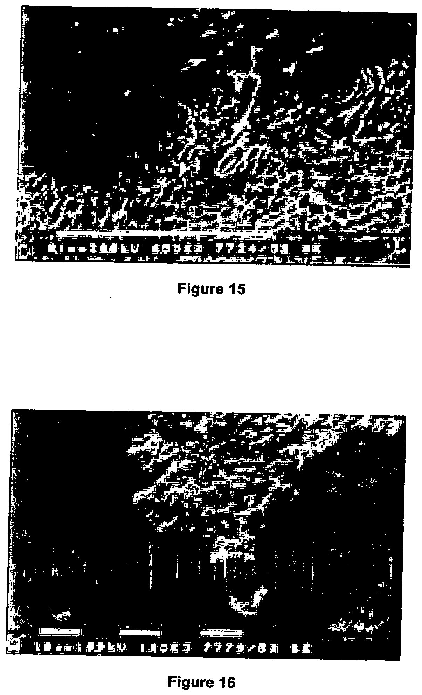

The most significant structure or change following the laser treatment is the loss of the characteristics of the crystal surface structure (prisms), due to fusion of the enamel (Figures 14, 15 and 16). These changes were not accompanied by the formation of cracks.

The formation of artificial cavities as a lesion always appeared in the control enamel windows (not treated); the formation in appearance thereof was completely inhibited in the windows that were laser-irradiated.

The microhardness profile of an enamel with laser and the profile of the enamel not treated with laser differ in the degree of hardness and the large increase in KHN, that is, a greater hardness, mat be clearly observed in the enamel of the windows treated with laser; likewise, a significant decrease KHN (lower hardness) is observed in the control enamel of the untreated windows.

It seems to be acceptable that the hardness values measured are proportional to the enamel mineral content; and, from the profiles found, we may assume that the modifications in the enamel permeability may play a significant role in these processes, which have already been widely discussed in the literature by various authors ([32], [33], [34] and [35]).

The effect presented is a combination of the change in permeability and the increase in the intrinsic resistance to the acid in the solution. Therefore, the decrease in the solubility of the enamel treated with the Nd:YAG laser under the conditions applied eliminates the pores or access spaces to deeper areas, thereby preventing ionic exchange between these areas and the de-mineralising solution (which produces the artificial cavity lesions). For all these reasons, it prevents and avoids the formation of cavity lesions.

**EXAMPLE 2. Preventive treatment for dental cavities. Study of the physical-mechanical effects of the Neodymium Yag laser upon being applied on the human dental surface or enamel.**

Nature and processing of the sample

A total sample of 460 healthy teeth was used in this study, whereof 230 teeth were randomly selected for the measurements, and the other 230 were used as a control sample. As in the preceding example, they were carefully selected to ensure that there were no lesions that might mask the effects of the treatment.

The teeth selected for the measurements were cleaned with 12% sodium hypoclhorite for 1 hour in order to eliminate the organic matter from the surface. Subsequently, they were rinsed with distilled water and their enamel crowns were cut and separated from the roots.

From the 230 teeth, a random sample of 150 crowns was used for the microhardness measurements and the other sample, composed of 80 crowns, was used for the permselectivity and permeability studies. The teeth in the hardness measurement sample were polished perpendicularly to the direction of the prisms (parallel to the surface) with polishing paper in a Buehler Motopol (polishing machine (Germany), in order to obtain a small plateau and, subsequently, serially polish them with 15 m$\mu$, 6 m$\mu$ and 1 m$\mu$ of Buehler abrasive diameter (Germany) and, later, with Buehler Metadi diamond spray (Germany) on a Buehler polishing cloth (Germany).

The teeth in the permeability study sample were embedded in epoxy resins and subsequently polished. Later, an 800- m$\mu$ section was cut using a Buehler Isometo low-speed saw or cutter (Germany) and mounted on a concentration of cells.

Laser irradiation conditions:

The 230 teeth selected for the measurement sample were subjected to irradiation with a DCR-2 Nd:YAG Laboratory Laser System from Quanta-Ray (United Kingdom). The following parameters were used for the laser irradi-

ation:

- Density: 4 J / mm$^2$.
- Frequency: 1 kHz.
- Pulse energy: 2 mJ / pulse.
- Spot size: 5 mm.
- Exposure time: 2 s.
- Peak power: 120 kW.
- Pulse width: 110 ns.
- Average energy: 13 W.
- Total energy per application: 26 J.

Post-treatment measurements:

*Microhardness measurement:*

In order to measure the microhardness, a Knoop diamond under a 50-g load was used in a Matsuzawa MTH-I machine (Japan). The Knoop hardness values were calculated on the basis of the length of each depression in the diamond, using the equation:

$$KHN - 14,230 \; X \; F/L^2$$

where F is the force applied in grams and L is the length of the depression produced by the diamond, measured in microns.
20 depressions were made on the plateau of each tooth at 150 m$\mu$, separated by regular space intervals. The hardness measurements on the enamel treated with laser were performed by the same operator and close to the depressions made on the untreated enamel, in order to minimise the experimental error.

*Permselectivity studies:*

In all the experiments, the electromagnetic field (e.m.f.) was measured in a concentration of cells of the type:

| Ref. electr. | // | Solution 1 | / | Membrane-enamel | / | Solution 2 | // | Ref. electr. |
| Ag +/AgC | // | | / | | | / | // | Ag+/AgC. |

where the double vertical lines indicate the location of the Clk convergence points. The potential developed through the membranes was measured with a Hipotest high-impedance recording potentiometer, model PE-W (Belgium).
All the solutions were prepared with analytical reagent-grade salts and distilled water. 25 of them were buffered with sodium phosphate at pH = 7.02 and the influence of the buffer ions on the e.m.f. was discarded.
The basis for the calculation is the theory of Teorell-Meyer-Sievers (TMS theory), which describes the transport of ions through a charged porous membrane the faces of which are in equilibrium with a solution of the same electrolyte 25. The total membrane potential E is calculated as follows:

$$E = \frac{R \cdot T}{F}\left[U \cdot \ln\left(\frac{\sqrt{X^2 + 4a_2^2} + U \cdot X}{\sqrt{X^2 + 4a_1^2} + U \cdot X}\right) + \ln\left(\frac{a_2}{a_1}\right) \cdot \left(\frac{\sqrt{X^2 + 4a_1^2} + X}{\sqrt{X^2 + 4a_2^2} + X}\right)\right].$$

where R is the gas constant, F is the Faraday constant, U = (D - 1 / D + 1), a and X are the membrane charge, X and D are determined by applying an interactive minimum adjustment method.

Results of the treatment:

The potentials of the membranes not treated with laser were positive and became more positive when the concentration of KCl increased. When the enamel membranes are treated with laser, the membrane potentials become more positive than those measured in the natural membranes of the control sample, indicating that the permselectivity of the enamel membranes is modified by the laser radiation. The microhardness values obtained in the control sample of a healthy enamel not treated with laser ranged between 340 and 388; these values agree with those previously published by other authors ([36] and [37]).

In all the teeth in the sample we observe that the Knoop hardness increases when measured after the laser was applied. These increases were significant and always occurred when their values were connected and related to the initial hardness values of the corresponding enamel prior to being treated. We observe and show that the high initial hardness values correspond to a given increase in hardness; and a lower initial hardness corresponds to a big increase in hardness following the laser radiation.

**EXAMPLE 3. Preventive treatment for dental caries. Study of the morphological effects of the Neodymium Yag laser upon being applied on the human dental surface or enamel.**

Nature and processing of the sample.

In order to perform this study, a sample of 350 human teeth extracted for orthodontic reasons was used. They were carefully selected, in order to ensure that there were no lesions that might mask the morphological effects for the treatment.

From the total teeth selected, a random sample of 250 teeth were treated with laser on the labial or vestibular surface, and the other 100 teeth were used as a control sample. The 250 teeth in the sample to be treated with laser were fixed with 2.5% glutaraldehyde, in 0.1 M sodium phosphate buffer (pH = 7.02), at a temperature of 4°C for 12 hours.

Subsequently, they were washed in the same buffer, with 10-minute baths each, and, later, with distilled water. Thereafter, the teeth were cleaned with 12% sodium hypochlorite for 1 hour, in order to eliminate the organic matter on the surface and, finally, they were etched with 0.5 M orthophosphoric acid for 1 minute and rinsed abundant distilled water.

Laser irradiation conditions:

The 250 teeth etched with acid were subjected to irradiation with a DCR-2 Nd:YAG Laboratory Laser System for Quanta-Ray (United Kingdom).

The following parameters were used for the laser irradiation:

- Density: 4 J / mm$^2$.
- Frequency: 1 kHz.
- Pulse energy: 2 mJ / pulse.
- Spot size: 5 mm.
- Exposure time: 2 s.
- Peak power: 120 kW.
- Pulse width: 110 ns.
- Average energy: 13 W.
- Total energy per application: 26 J.

A Siemens LGK 7672 Helium-Neo laser (Meinchen, Germany) was used to visualise and locate the Nd:YAG laser beam in the treated area.

Post-treatment measurements

*Observations with the scanning electron microscope:*

The teeth were processed in accordance with the conventional SEM method, and coated or coated with gold with a Bio-Rad sprayer, model SC 5000 (Netherlands). The entire sample of teeth was examined with a Philips 515 scanning electron microscope (Netherlands) at 5 kV.

*Roughness measurements:*

The degree of roughness of the enamel surface was measured with a Mituyo Suftest 201 surface roughness meter (Japan). The measurements were performed on teeth treated with laser and control teeth, using a cut-off value (lambda c) of 0.25 mm and an evaluation length (5 x lambda c) of 1.25 mm. Several roughness profile parameters were obtained. The most significant were Ra (lower arithmetic deviation of the roughness profiles) and Pc (peak count); defined as follows:

$$R_a = \frac{1}{lm} \int_0^{lm} |f(x)|dx$$

- Ra is the lower arithmetic deviation of the absolute values of the profiles deviated from the central line with the evaluation of lenght lm
- The roughness profiles are given as Y=f(x), with the "x" axis for the centre of the line, and the "y" axis in the direction of the magnification of the vertical line.
- Pc is the number of peak profiles (maximum) per unit of length. In order to determine the peak count, two parallel lines are drawn at a given level, below and above the central line over the longitudinal evaluation. A peak profile is defined as the proportion of the projecting profile that produces above the upper line, at the two adjacent points of intersection of the profile with the lower line.

In this study, a level of 1.3 m$\mu$ was used to determine the peak count.

Results of the treatment

The surface of human dental enamel normally shows a somewhat smooth area, which may be observed under the SEM.

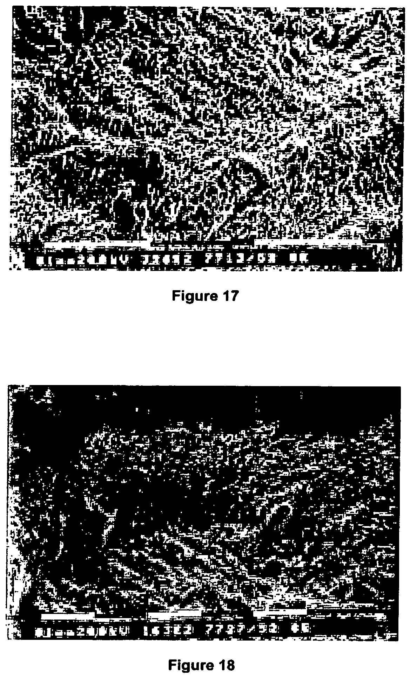

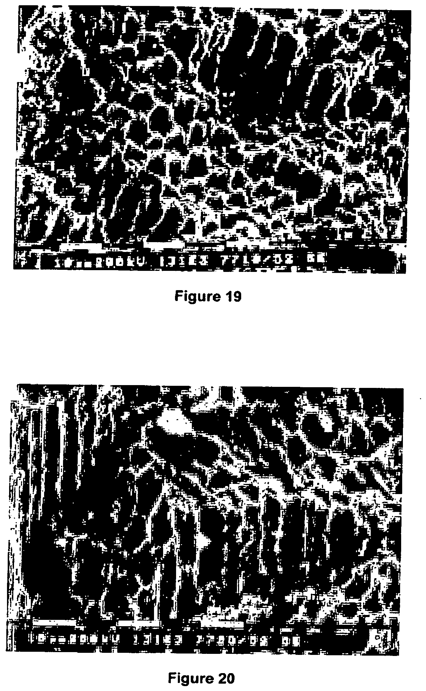

In healthy human dental enamel not subjected to any treatment, the most significant structural reliefs are represented by the perikymata (21), which are regularly distributed as multiple small waves (Figures 17, 18, 19 and 20).

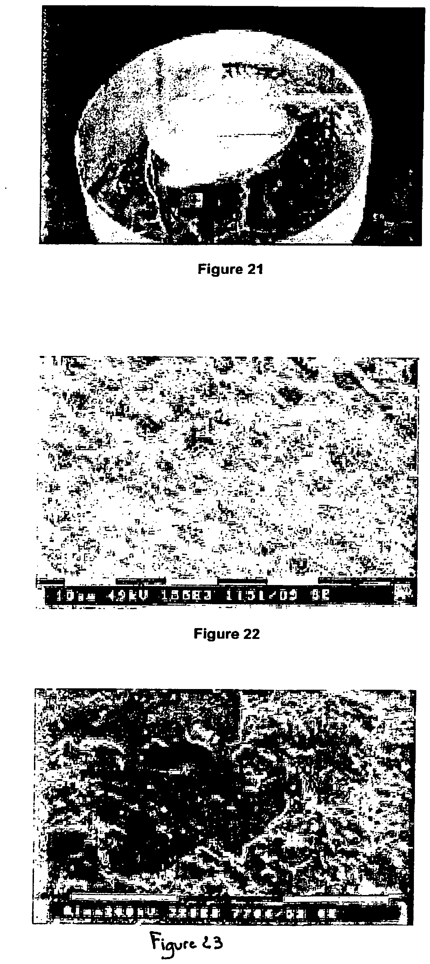

A typical roughness measurement of this surface is that obtained on the labial surface of a healthy human premolar tooth with the following parameters: Ra = 1.6 $\pm$0.1 m$\mu$, and Pc = 0 cm$^{-1}$ (Figure 21).

Under scanning electron microscopy, the characteristic morphology of the hydroxyapatite prisms and the interprismatic matter on an enamel surface etched with acid are clearly observed, and the polyedric structure of the prisms is also sharply seen.

The roughness of the enamel etched with acid greatly increases. This increase is represented by a rise in the Ra and Pc parameters as compared to the values corresponding to healthy non-etched enamel.

In all the samples etched with acid, it was observed that Ra ranged from 1.8 to 2.3 m$\mu$, and Pc ranged from 35 to 50 cm$^{-1}$. The effects caused by application of the laser under certain parameters on the surface of an enamel previously etched with acid and observed under the SEM reveals the loss of the prisms' characteristic structure due to the surface fusion of the dental enamel (Figure 22). This structural change is directly related to the general decrease in the rough surface (Figure 23), taking into consideration the significant fact that the enamel etched with acid was evaluated by measuring its profile. The roughness parameter values obtained following the treatment with laser range between 1.2 and 1.6 m$\mu$ for Ra, and between 0 and 5 cm$^{-1}$ for Pc.

The comparative values of the roughness parameters obtained are:

| Control sample of teeth not etched and not treated with laser | Treated sample of etched teeth, not treated with laser | Treated sample of etched teeth, treated with laser |
|---|---|---|
| (a) | (b) | (c) |
| Ra: 1,6 $\pm$ 0,1m$\mu$ | 1,8 a 2,3 m$\mu$ | 1,2 a 1,6 m$\mu$ |
| Pc: 0 cm$^{-1}$ | 35 a 50 cm$^{-1}$ | 0 a 5 cm$^{-1}$ |

The sample of teeth etched with acid and treated with laser exhibited a smoother surface, that is, with a lower roughness than the control sample.

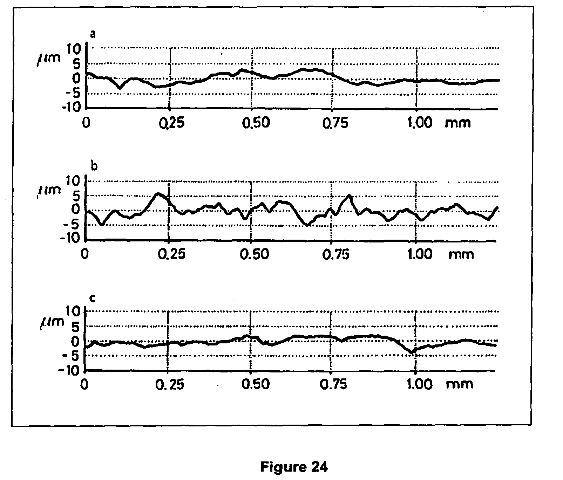

Figure 24 shows the roughness profiles obtained on the vestibular face of the enamel surface of different tooth samples: a) Roughness profiles of the control dental sample, teeth not etched with acid and not treated with laser. b) Roughness profiles of the treated dental sample, teeth etched with acid and not treated with laser. c) Roughness profiles of the treated dental sample, teeth etched with acid and subsequently treat-ed with Nd:YAG laser.

We observe the greatest increase in the roughness of the enamel following etching with acid (b) and a decreased in or loss of roughness following application of the laser (c), the latter being even lower than in the control sample (a). This indicates that the absorption of laser energy was sufficient to modify the structure of the enamel surface etched with acid, without the need to coat it with absorbent colouring substances.

**EXAMPLE 4. Corrective treatment for dental cavities. Study of new technology with Neodymium:YAG laser and mineral hydroxyapatite as a new cementing and restorative material for human teeth.**

Nature and processing of the sample:

In order to perform the process of this invention, a sample composed of 350 human teeth was selected. The selection criterion was that they show dental cavities (250teeth) or crown fractures (100 remaining ones).

In order to eliminate the decayed tissue, the interior of each tooth with cavities was treated with Komet diamond drills and tungsten carbide (Germany); located on a water-cooled Kavo turbine (Germany). The entire inside of the cavities and the edges of the preparations were etched with 36% orthophosphoric acid, the enamel for 30 seconds and the dentin for 15 seconds; subsequently, they were washed with abundant water and dried.

The cavities were made, prepared and treated by the same operator in order to minimize the potential experimental error. All the teeth in the sample were fixed with 2.5% glutaraldehyde in 0.1 M buffered sodium phosphate (pH = 7.02), at 4°C, for 12 hours. Subsequently, they were washed with the same buffer in 3 baths, 10 minutes each, and, later, with distilled water.

Post-treatment measurements:

Hardness measurements were performed on cross sections of the biomaterial cements applied with the laser, in order to verify whether this hardness is similar to that of healthy dental enamel. The effects generated on the biomaterial by the Nd:YAG laser irradiation were studied in terms of the microhardness and permselectivity thereof, being evaluated by means of Knoop studies and by Teorell-Meyer-Sievers theory, respectively.

The treated teeth were studied with the scanning electron miscroscope (SEM) on the entire outer surface of the inner junction area between the cement biomaterial and the tooth (enamel and dentin). Roughness measurements were also performed after applying the biomaterial with the laser, on the cementing and restorative surfaces, as well as on the adjacent enamel surface.

The total sample was randomly distributed in the following manner: 80 teeth with cavities and 25 teeth with fractures were used for the hardness study and the observation in the SEM analyser; another 80 decayed teeth and 30 fractures teeth were used for the microhardness and permselectivity studies; and, as the final group, 90 teeth with cavities and 45 teeth with fractures were used for the roughness measurements and the observations with the SEM. The teeth in the sample for the hardness measurements were polished with a specific paper in a Buehler Motopol 8 polishing machine (Germany). Subsequently, they were serially polished with 15 m$\mu$, 6 m$\mu$ and 1 m$\mu$ of Buehler abrasive diamond (Germany) and, later, with Buehler Metadi diamond spray (Germany) on a Buehler polishing cloth (Germany).

The teeth in the sample for the permeability studies were embedded in an epoxy resin and polished; finally, an 800- m$\mu$ section was cut using a Buehler Isometo low-speed saw or cutter (Germany) and mounted on a concentration of cells.

Composition and application of the biomaterial: hydroxyapatite paste

The composition of the biomaterial paste applied on the teeth was the following: dense, powdered, micronized mineral hydroxyapatite, mixed with gelatin in order to form a very consistent, thick paste. The density of the paste was controlled by an Isaka RX-10 densiometer (Japan).

This hydroxyapatite paste was used as a cementing and restorative material for teeth; it was applied with an Aesculap 1057 condenser-moulder instrument (Germany), by layers, each layer being approximately 1 mm thick.

Nd:YAG laser irradiation

During the application thereof, the hydroxyapatite was subjected to irradiation with a DCR-2 Nd:YAG Laboratory Laser System from Quanta-Ray (United Kingdom). Moreover, each layer of hydroxyapatite was fused to the preparation walls, dentin and/or dental enamel with the same laser.

*Nd:YAG laser irradiation conditions*

The following Nd:YAG laser irradiation conditions were applied:

- Density: 28 J / mm$^2$.
- Frequency: 5 kHz.
- Pulse energy: 8 mJ / pulse.

- Spot size: 3 mm.
- Exposure time: 5 s.
- Peak power: 72 kW.
- Pulse width: 120 ns.
- Average energy: 40 W.
- Total energy per application: 200 J.

A Siemens LGK 7672 Helium-Neon laser (Meinchen, Germany) was used to visualise and locate the laser beam in the treated area.

Post-treatment measurements

*Hardness measurements:*

They were performed with a Matsuzawa MTH-1 hardness meter (Japan), using a diamond tip under a 10-g load. The KDN hardness numbers were calculated by developing the following equation:

$$KHN = 14,230 \times F/L\ 2$$

where L is the length of each depression (entry) of the diamond in microns and F is applied force in grams.

*Microhardness measurements:*

A Knoop diamond under a 50-g load in a Matsuzawa MTH-1 microhardness meter (Japan) was used. 20 depressions were made at 150 $\mu$, separated at regular space intervals (equidistant) on the hydroxyapatite plateau in each tooth. The microhardness measurements on the cementing and restorative material, in this case mineral hydroxyapatite, and on the adjacent dental enamel, were performed by the same operator in order to minimize the experimental error.

*Permselectivity measurements:*

All the solutions for the permselectivity measurements were prepared using reagent-grade salts and distilled water. They were buffered with sodium phosphate at pH = 7.02 and the influence of the buffer ions on the electromagnetic field (e.m.f.) was discarded.
The basis for the calculation is the Teorell-Meyer-Sievers theory (TMS), which describes the transport of ions through a charged porous membrane the faces of which are in equilibrium with a solution of the same electrolyte.
The total membrane potential, E, is calculated as:

$$E = \frac{R \cdot T}{F}\left[ U \cdot ln\left(\frac{\sqrt{X^2 + 4a_2^2} + U \cdot X}{\sqrt{X^2 + 4a_1^2} + U \cdot X}\right) + ln\left(\frac{a_2}{a_1}\right) \cdot \left(\frac{\sqrt{X^2 + 4a_1^2} + X}{\sqrt{X^2 + 4a_2^2} + X}\right)\right]$$

where R is the gas constant, F is the Faraday constant, U = (D - 1 ÷ d + 1), a and X are the membrane charge, X and D are determined by applying an interactive minimum adjustment method.

*Observations with the scanning electron microscope:*

After being treated with the biomaterial and the Nd:YAG laser, the teeth in the sample were widely studied by the scanning electron microscope analyser. The teeth in the sample were processed in accordance with the conventional method and coated with gold, using a Bio-Rad metalliser, model SC 5000 (Netherlands). Moreover, these teeth were subsequently examined with a Philips 515 SEM (Netherlands) at 20 kV.

*Roughness measurements:*

The degree of roughness of the surface of the cementing and restorative mineral hydroxyapatite was measured with a Mituyo Suftest 201 roughness and surface roughness meter (Japan). A cut-off value (lambda c) of 0.25 mm and an evaluation length (5 x lambda c) of 1.25 mm were used.

The Ra and Pc roughness parameters were used. The most significant were Ra (lower arithmetic deviation of the roughness profiles) and Pc (peak count); defined as follows:

$$R_a = \frac{1}{lm} \int_0^{lm} |f(x)| dx$$

- Ra is the lower arithmetic of the absolute values of the profiles deviated from the central line with the evaluation of length lm.
- The roughness profiles are given as Y = f(x), with the "x" axis for the centre of the line, and the "y" axis in the direction of the magnification of the vertical line.
- Pc is the number of peak profiles (maxima) per unit of length. In order to determine the peak count, two parallel lines are drawn at a given level, below and above the central line over the longitudinal evaluation. A peak profile is defined as the proportion of the projecting profile that produces above the upper line, at the two points adjacent to the intersection between the profile and the lower line.

In this work, we have used a level of 1.3 m$\mu$ to determine the peak count. In order to study the teeth, a Zeiss polarised-light optical microscope (Germany) was also used.

Results of the treatment

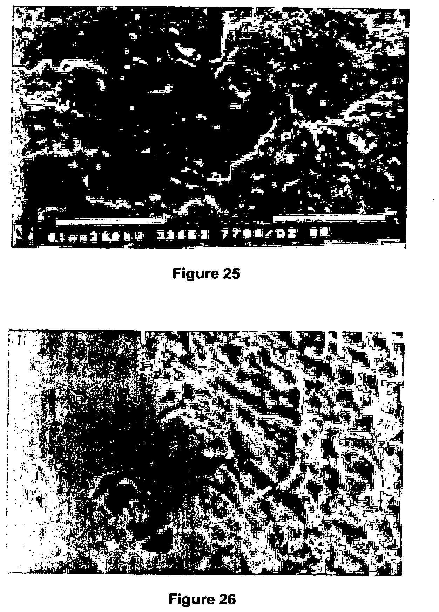

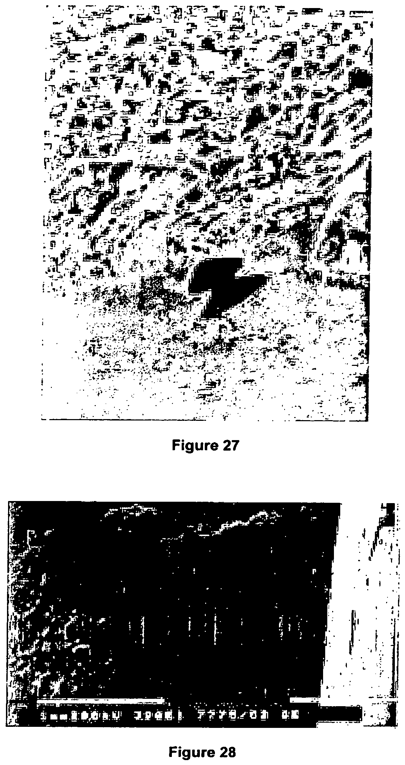

Mineral hydroxyapatite, fused with a Nd:YAG laser and used as a cementing and restorative material, presented a completely amorphous structure, without a dense, compact crystallographic distribution (Figures 25 and 26). The microfusion of mineral hydroxyapatite was not accompanied by the formation of cracks (Figures 27, 28 and 29). The hardness of mineral hydroxyapatite, measured in KDN in accordance with the KHN equation, is similar of that of healthy dental enamel. The permeability of mineral hydroxyapatite is null or practically non-existent.

The Knoop microhardness values of mineral hydroxyapatite obtained range between 335 and 380, and are within the range of microhardness values for healthy enamel, that is, between 340 and 388, the values published by other authors ([37], [38], [39], [40] and [41]).

The roughness measurement produced the parameters Ra = 1.4 to 1.8 m$\mu$ and Pc = 2 to 5 cm$^{-1}$, which, compared to those of healthy enamel, are equivalent and very similar. A typical roughness measurement for healthy enamel is that obtained on the labial surface of a healthy human premolar tooth, which has the parameters: Ra = 1.6 $\pm$ 0.1 m$\mu$, and Pc = 0 cm$^{-1}$.

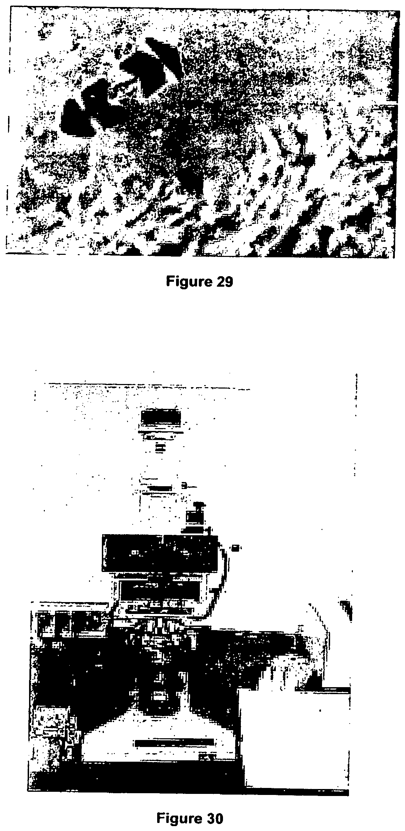

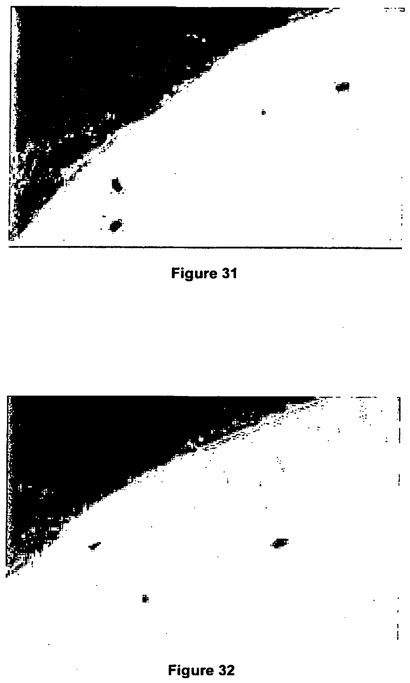

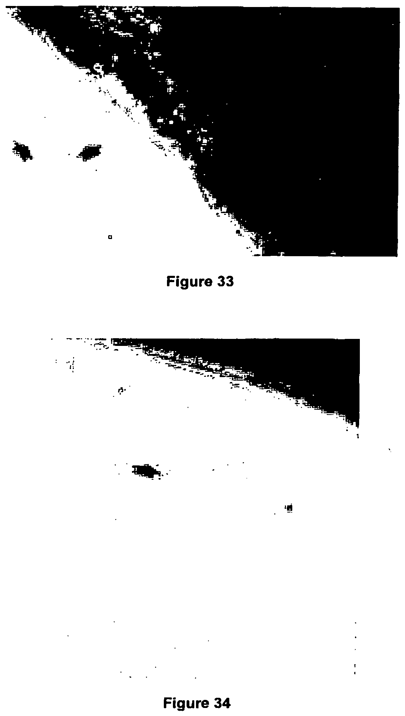

This indicates that the absorption of energy was sufficient to produce the microfusion of mineral hydroxyapatite without the need to use absorbent colouring substances (Figures 30 and 31). No residues of the excipient (gelatin) of the hydroxyapatite paste were found following the microfusion, which shows that, although it is the necessary carrier to obtain a pasta consistency, when it is applied on the cavity, said excipient is volatilised by the laser radiation, and the fused mineral hydroxyapatite remains fully compact (Figures 32 and 33).

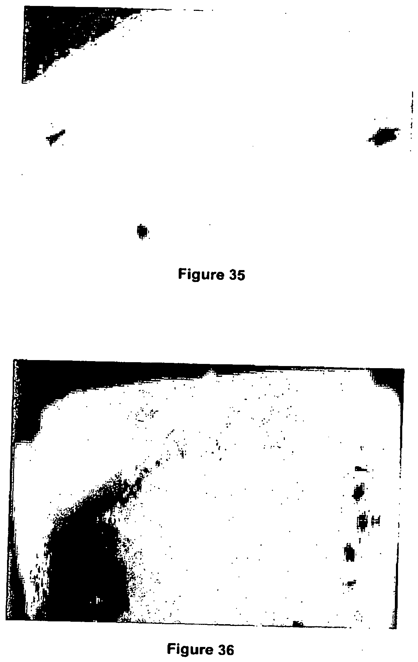

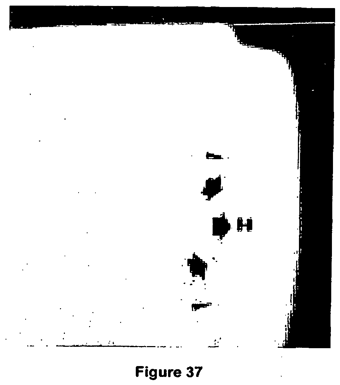

The junction of the dentin and dental enamel with the mineral hydroxyapatite takes place by the fusion of both parts (Figures 34, 35, 36 and 37), which confers a very high resistance, much higher than that of mechanical-retentive (amalgams) and adhesive (glass ionomers and composites) retentions.

The application of the laser radiation takes place with maximum energy, in a very short period of time; consequently, it does not cause thermal damage in soft tissues of in the dental pulp.

**BIBLIOGRAPHY**

[1] Zhang XZ, Anderson P, Dowker SE, Elliott JC. Optical profilometric study of changes in surface roughness of enamel during in vitro demineralization . Caries Res. 2000 Mar-Apr;34 (2): 164-74.

[2] Kawasaki K, Tanaka Y, Takagi O. Crystallographic analysis of demineralized human enamel treated by laser-irradiation or remineralization . Arch Oral Biol. 2000 Sep;45(9) :797-804.

[3] Margolis HC, Zhang YP, Lee CY, Kent RL Jr, Moreno EC. Kinetics of enamel demineralization in vitro. J Dent Res. 1999 Jul; 78 (7) :1326-35.

[4] Brookes SJ, Shore RC, Robinson C, Wood SR, Kirkham J. Copper ions inhibit the demineralisation of human enamel. Arch Oral Biol. 2003 Jan; 48 (1) : 25-30.

[5] Kuhar M, Cevc P, Schara M, Funduk N. In vitro permeability and scanning electron microscopy study of acid-etched and ground enamel surfaces protected with dental adhesive coating. J Oral Rehabil. 1999 Sep;26 (9) : 722-30.

[6] Turssi CP, Schiavoni RJ, Serra MC, Froner IC Permeability of enamel following light-activated power bleaching. Gen Dent. 2006 Sep-Oct ; 54 (5) : 323-6.

[7] Zanet CG, Arana-Chavez VE, Fava M. Scanning electron microscopy evaluation of the effect of etching agents on human enamel surface. J Clin Pediatr Dent. 2006 Spring;30 (3) :247-50.

[8] Pick RM. Lasers in dentistry: where we are today. Dent Today. 2000 Sep; 19 (9) : 50-3.

[9] Cernavin I, Hogan SP. The effects of the Nd: YAG laser on amalgam dental restorative material. Aust Dent J. 1999 Jun;44 (2) :98-102.

[10] Neev J, Nelson JS, Critelli M, McCullough JL, Cheung E, Carrasco WA, et al. Ablation of human nail by pulsed lasers. Lasers Surg Med. 1997; 21 (2) : 186-92.

[11] Yamada MK, Uo M, Ohkawa S, Akasaka T, Watari F. Three- dimensional topographic scanning electrón microscope and Raman spectroscopic analyses of the irradiation effect on teeth by Nd: YAG, Er: YAG, and CO (2) lasers. J Biomed Mater Res B Appl Biomater. 2004 Oct 15; 71 (1): 7-15.

[12] McCormack SM, Fried D, Featherstone JD, Glena RE, Seka W. Scanning electron microscope observations of CO2 laser effects on dental enamel. J Dent Res. 1995 Oct; 74 (10): 1702- 8.

[13] Anic I, Segovic S, Katanec D, Prskalo K, Naj zar-Fleger D. Scanning electrón microscopic study of dentin lased with argon, CO2, and Nd:YAG láser. J Endod. 1998 Feb; 24 (2) : 77- 81.

[14] McDavid VG, Cobb CM, Rapley JW, Glaros AG, Spencer P. Láser irradiation of bone : III. Long-term healing following treatment by CO2 and Nd: YAG lasers. J Periodontol. 2001 Feb; 72 (2) :174-82.

[15] Westerman GH, Hicks MJ, Flaitz CM, Powell GL, Blankenau RJ. Surface morphology of sound enamel after argon laser irradiation: an in vitro scanning electron microscopy study. J Clin Pediatr Dent. 1996 Fall; 21 (1): 55-9.

[16] Hsu CY, Jordán TH, Dederich DN, Wefel JS. Effects of low-energy C02 laser irradiation and the organic matrix on inhibition of enamel demineralization. J Dent Res. 2000 Sep; 79 (9): 1725-30.

[17] Aminzadeh A, Shahabi S, Walsh LJ. Raman spectroscopic studies of C02 laser-irradiated human dental enamel. Spectrochim Acta A Mol Biomol Spectrosc. 1999 Jun; 55A(6):1303-8.

[18] Bahar A, Tagomori S. The effect of normal pulsed Nd-YAG laser irradiation on pits and fissures in human teeth. Caries Res. 1994; 28 (6): 460-7.

[19] Gelskey SC, White JM, Gelskey DE, Kremers W. Vapor emissions resulting from Nd:YAG laser interaction with tooth structure. Dent Mater. 1998 Nov; 14 (6) : 453-7.

[20] Myaki SI, Watanabe IS, Eduardo Cde P, Issao M. Nd: YAG laser effects on the occlusal surface of premolars. Am J Dent. 1998 Jun; 11 (3): 103-5.

[21] Harris DM, White JM, Goodis H, Arcoria CJ, Simon J, Carpenter WM, et al. Selective ablation of surface enamel caries with a pulsed Nd: YAG dental láser. Lasers Surg Med. 2002;30 (5) :342-50.

[22] Yamada Y, Hossain M, Kawanaka T, Kinoshita J, Matsumoto K. Removal effects of the Nd:YAG láser and Carisolv on carious dentin. J Clin Laser Med Surg. 2000 Oct; 18 (5) : 241- 5.

[23] Depraet FJ, De Bruyne MA, De Moor RJ. The sealing ability of an epoxy resin root canal sealer after Nd:YAG laser irradiation of the root canal. Int Endod J. 2005 May;38 (5) :302-9.

[24] Pick RM. Lasers in dentistry: where we are today. Dent Today. 2000 Sep; 19 (9) : 50-3. No abstract available.

[25] Ferro D, Barinov SM, Rau JV, Teghil R, Latini A. Calcium phosphate and fluorinated calcium phosphate coatings on titanium deposited by Nd:YAG laser at a high fluence. Biomaterials. 2005 Mar.; 26 (7): 805-12.

[26] Kobayashi CA, Fujishima A, Miyazaki T, Kimura Y, Matsumoto K, Osada T, et al. Effect of Nd: YAG laser irradiation on shear bond strength of glass-Ionomer luting cement to dentin surface. Int J Prosthodont. 2003 Sep-Oct;16 (5) :493-8.

[27] Pioch T, Moller D, Staehle HJ, Hoppe W. Solubility of enamel and synthetic hydroxyl apatite on irradiation. Dtsch Zahnarztl Z. 1991 Jun.; 46 (6): 413-5. German.

[28] Quintana E, Márquez F, Roca I, Torres V, Salgado J. Some morphologic changes induced by Nd: YAG laser on the noncoated enamel surface: a scanning electron microscopy study. Lasers Surg Med. 1992; 12 (2): 131-6.

[29] Swift EJ Jr, Edwards GS, Perdigao J, Thompson JY, Nunes MF, Ruddell DE, et al. Free-electron laser etching of dental enamel. J Dent. 2001 Jul; 29 (5): 347-53.

[30] Sazak H, Turkmen C, Gunday M. Effects of Nd: YAG laser, air-abrasion and acid-etching on human enamel and dentin. Oper Dent. 2001 Sep-Oct; 26 (5): 476-81.

[31] Zentner A, Duschner H. Structural changes of acid etched enamel examined under confocal laser scanning microscope. J Orofac Orthop. 1996 Aug; 57 (4): 202-9. English, German.

[32] Korytnicki D, Mayer MP, Daronch M, Singer Jda M, Grande RH. Effects of Nd:YAG laser on enamel microhardness and dental plaque composition: an in situ study. Photomed Laser Surg. 2006 Feb; 24 (1): 59-63.

[33] Kuramoto Junior M, Matson E, Turbino ML, Marques RA. Microhardness of Nd:YAG laser irradiated enamel surfaces. Braz Dent J. 2001; 12 (1): 31-3.

[34] Kielbassa AM, Wrbas KT, Schulte-Monting J, Hellwig E. Correlation of transversal microradiography and microhardness on in situ-induced demineralization in irradiated and nonirradiated human dental enamel. Aren Oral Biol. 1999 Mar; 44 (3): 243-51.

[35] Salama SN, Kinawi NA. X-ray study and microhardness data of some dental enamel species. Biomaterials. 1989 Apr.; 10 (3): 209-12.

[36] Fosse G, Rosengren B, Skaale S, Leknes K, Wulff L. An in vivo method for microhardness measurements on human teeth. Scand J Dent Res. 1986 Feb. 94 (1): 27-37.

[37] Demetriades A, Koulourides T. Experimental studies of dental caries. Measurements of enamel microhardness. Stomatologia (Athenai) . 1969 Mar-Apr. 26 (2): 69-78. Greek, Modern.

[38] Márquez F, Quintana E, Roca I, Salgado J. Physical-mechanical effects of Nd:YAG laser on the surface of sound dental enamel. Biomaterials. 1993; 14 (4): 313-6.

[39] Danilina TF, Bagmutov VP, Slavskii Iul. The microhardness of dental tissues as an index of their normal functional stability and in pathological states. Stomatologiia (Mosk). 1998; 77 (3): 9-11. Russian.

[40] Klinger HG, Wiedemann W, Docos E. Mechanical properties and acid solubility of the dental surface. I. Microhardness. Dtsch Zahnarztl Z. 1980 Aug; 35 (8): 823-7. German.

[41] Florin R, Herrmann C, Bernhardt W. Measuring microhardness of laser exposed tooth surface. Stomatol DDR. 1990 Feb. 40 (2): 49-51. German.

## Claims

1. A system for laser treatment of dental caries comprising:

   at least one biomaterial layer with a content of mineral hydroxyapatite which is arranged to be applied in a dental surface or enamel; and
   a Nd:YAG laser arranged to irradiate the biomaterial layer with a variable and regulated optical focusing of the laser beam from a maximum focusing to a complete defocusing until the biomaterial fuses with the surface;
   Wherein the Nd:YAG laser is arranged to perform the irradiation according to the following parameters: (a) density comprised between 3 and 30 $J/mm^2$; (b) frequency comprised between 1 and 10 KHz; (c) pulse energy comprised between 1 and 10 mJ/pulse; (d) spot size comprised between 1 and 6 mm; (e) exposure time comprised between 1 and 6 seconds; (f) peak power comprised between 70 and 125 kW; (g) pulse width comprised between 100 and 130 ns; (h) average energy comprised between 10 and 50 W; and (i) total energy per application comprised between 15 and 220 J.

2. The system of claim 1 wherein the content of mineral hydroxyapatite in the biomaterial is of at least 75% in weight.

3. The system of any of claims 1 or 2 wherein the biomaterial layers have a thickness of between 0.5 and 2 mm.

4. The system of claim 3 wherein the biomaterial layers have a thickness of 1 mm.

## Patentansprüche

1. Ein System zur Laserbehandlung von Zahnkaries, bestehend aus:

   mindestens eine Biomaterialschicht mit einem Gehalt an mineralischem Hydroxylapatit, die so angeordnet ist, dass sie in eine Zahnoberfläche oder in den Zahnschmelz eingebracht werden kann; und
   einen Nd:YAG-Laser, der so angeordnet ist, dass die Biomaterialschicht mit einer variablen und geregelten optischen Fokussierung des Laserstrahls von einer maximalen Fokussierung bis zu einer vollständigen Defokussierung bestrahlt wird, bis das Biomaterial mit der Oberfläche verschmilzt;
   worin der Nd:YAG-Laser so angeordnet ist, dass er die Bestrahlung gemäß den folgenden Parametern durchführt: (a) Dichte zwischen 3 und 30 $J/mm^2$; (b) Frequenz zwischen 1 und 10 KHz; (c) Impulsenergie zwischen 1 und 10 mJ/Impuls; (d) Spotgröße zwischen 1 und 6 mm; (e) Belichtungszeit zwischen 1 und 6 Sekunden; (f) Spitzenleistung zwischen 70 und 125 kW; (g) Impulsbreite zwischen 100 und 130 ns; (h) Durchschnittsenergie zwischen 10 und 50 W; und (i) Gesamtenergie pro Anwendung zwischen 15 und 220 J.

2. Das System nach Anspruch 1, bei dem der Gehalt an mineralischem Hydroxylapatit im Biomaterial mindestens 75 Gew.-% beträgt.

**3.** Das System nach einem der Ansprüche 1 oder 2, bei dem die Biomaterialschichten eine Dicke zwischen 0,5 und 2 mm haben.

**4.** Das System nach Anspruch 3, bei dem die Biomaterialschichten eine Dicke von 1 mm haben.

**Revendications**

**1.** Un système de traitement au laser des caries dentaires comprenant:

au moins une couche de biomatériau ayant une teneur en hydroxyapatite minérale qui est prévue pour être appliquée sur une surface dentaire ou sur l'émail ; et
un laser Nd:YAG conçu pour irradier la couche de biomatériau avec une focalisation optique variable et régulée du faisceau laser, allant d'une focalisation maximale à une défocalisation complète jusqu'à ce que le biomatériau fusionne avec la surface;
dans lequel le laser Nd:YAG est agencé pour effectuer l'irradiation selon les paramètres suivants : (a) densité comprise entre 3 et 30 J/mm$^2$ ; (b) fréquence comprise entre 1 et 10 KHz ; (c) énergie d'impulsion comprise entre 1 et 10 mJ/impulsion ; (d) taille du spot comprise entre 1 et 6 mm ; (e) temps d'exposition compris entre 1 et 6 secondes ; (f) puissance de crête comprise entre 70 et 125 kW ; (g) largeur d'impulsion comprise entre 100 et 130 ns ; (h) énergie moyenne comprise entre 10 et 50 W ; et (i) énergie totale par application comprise entre 15 et 220 J.

**2.** Le système de la revendication 1, dans lequel la teneur en hydroxyapatite minérale dans le biomatériau est d'au moins 75% en poids.

**3.** Le système d'une des revendications 1 ou 2 dans lequel les couches de biomatériaux ont une épaisseur comprise entre 0,5 et 2 mm.

**4.** Le système de la revendication 3, dans lequel les couches de biomatériaux ont une épaisseur de 1 mm.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

**Figure 21**

**Figure 22**

Figure 23

**Figure 24**

Figure 25

Figure 26

Figure 27

Figure 28

**Figure 29**

**Figure 30**

**Figure 31**

**Figure 32**

Figure 33

Figure 34

Figure 35

Figure 36

Figure 37

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0392951 A2
- DE 4339779 A1

### Non-patent literature cited in the description

- **ZHANG XZ ; ANDERSON P ; DOWKER SE ; ELLIOTT JC.** Optical profilometric study of changes in surface roughness of enamel during in vitro demineralization. *Caries Res.,* March 2000, vol. 34 (2), 164-74
- **KAWASAKI K ; TANAKA Y ; TAKAGI O.** Crystallographic analysis of demineralized human enamel treated by laser- irradiation or remineralization. *Arch Oral Biol.,* September 2000, vol. 45 (9), 797-804
- **MARGOLIS HC ; ZHANG YP ; LEE CY ; KENT RL JR ; MORENO EC.** Kinetics of enamel demineralization in vitro. *J Dent Res.,* July 1999, vol. 78 (7), 1326-35
- **BROOKES SJ ; SHORE RC ; ROBINSON C ; WOOD SR ; KIRKHAM J.** Copper ions inhibit the demineralisation of human enamel. *Arch Oral Biol.,* January 2003, vol. 48 (1), 25-30
- **KUHAR M ; CEVC P ; SCHARA M ; FUNDUK N.** In vitro permeability and scanning electron microscopy study of acid-etched and ground enamel surfaces protected with dental adhesive coating. *J Oral Rehabil.,* September 1999, vol. 26 (9), 722-30
- **TURSSI CP ; SCHIAVONI RJ ; SERRA MC ; FRONER IC.** Permeability of enamel following light-activated power bleaching. *Gen Dent.,* September 2006, vol. 54 (5), 323-6
- Scanning electron microscopy evaluation of the effect of etching agents on human enamel surface. **ZANET CG ; ARANA-CHAVEZ VE ; FAVA M.** J Clin Pediatr Dent. Spring, 2006, vol. 30, 247-50
- **PICK RM.** Lasers in dentistry: where we are today. *Dent Today,* September 2000, vol. 19 (9), 50-3
- **CERNAVIN I ; HOGAN SP.** The effects of the Nd: YAG laser on amalgam dental restorative material. *Aust Dent J.,* June 1999, vol. 44 (2), 98-102
- **NEEV J ; NELSON JS ; CRITELLI M ; MCCULLOUGH JL ; CHEUNG E ; CARRASCO WA et al.** Ablation of human nail by pulsed lasers. *Lasers Surg Med.,* 1997, vol. 21 (2), 186-92
- **YAMADA MK ; UO M ; OHKAWA S ; AKASAKA T ; WATARI F.** Three- dimensional topographic scanning electrón microscope and Raman spectroscopic analyses of the irradiation effect on teeth by Nd: YAG, Er: YAG, and CO (2) lasers. *J Biomed Mater Res B Appl Biomater,* 15 October 2004, vol. 71 (1), 7-15
- **MCCORMACK SM ; FRIED D ; FEATHERSTONE JD ; GLENA RE ; SEKA W.** Scanning electron microscope observations of CO2 laser effects on dental enamel. *J Dent Res.,* October 1995, vol. 74 (10), 1702-8
- **ANIC I ; SEGOVIC S ; KATANEC D ; PRSKALO K ; NAJ ZAR-FLEGER D.** Scanning electrón microscopic study of dentin lased with argon, CO2, and Nd:YAG láser. *J Endod.,* February 1998, vol. 24 (2), 77-81
- **MCDAVID VG ; COBB CM ; RAPLEY JW ; GLAROS AG ; SPENCER P.** Láser irradiation of bone : III. Long-term healing following treatment by CO2 and Nd: YAG lasers. *J Periodontol.,* February 2001, vol. 72 (2), 174-82
- Surface morphology of sound enamel after argon laser irradiation: an in vitro scanning electron microscopy study. **WESTERMAN GH ; HICKS MJ ; FLAITZ CM ; POWELL GL ; BLANKENAU RJ.** J Clin Pediatr Dent. Fall, 1996, vol. 21, 55-9
- **HSU CY ; JORDÁN TH ; DEDERICH DN ; WEFEL JS.** Effects of low-energy C02 laser irradiation and the organic matrix on inhibition of enamel demineralization. *J Dent Res.,* September 2000, vol. 79 (9), 1725-30
- **AMINZADEH A ; SHAHABI S ; WALSH LJ.** Raman spectroscopic studies of C02 laser-irradiated human dental enamel. *Spectrochim Acta A Mol Biomol Spectrosc.,* June 1999, vol. 55A (6), 1303-8
- **BAHAR A ; TAGOMORI S.** The effect of normal pulsed Nd-YAG laser irradiation on pits and fissures in human teeth. *Caries Res.,* 1994, vol. 28 (6), 460-7
- **GELSKEY SC ; WHITE JM ; GELSKEY DE ; KREMERS W.** Vapor emissions resulting from Nd:YAG laser interaction with tooth structure. *Dent Mater.,* November 1998, vol. 14 (6), 453-7

- **MYAKI SI ; WATANABE IS ; EDUARDO CDE P ; ISSAO M.** Nd: YAG laser effects on the occlusal surface of premolars. *Am J Dent.,* June 1998, vol. 11 (3), 103-5
- **HARRIS DM ; WHITE JM ; GOODIS H ; ARCORIA CJ ; SIMON J ; CARPENTER WM et al.** Selective ablation of surface enamel caries with a pulsed Nd: YAG dental láser. *Lasers Surg Med.,* 2002, vol. 30 (5), 342-50
- **YAMADA Y ; HOSSAIN M ; KAWANAKA T ; KI-NOSHITA J ; MATSUMOTO K.** Removal effects of the Nd:YAG láser and Carisolv on carious dentin. *J Clin Laser Med Surg.,* October 2000, vol. 18 (5), 241-5
- **DEPRAET FJ ; DE BRUYNE MA ; DE MOOR RJ.** The sealing ability of an epoxy resin root canal sealer after Nd:YAG laser irradiation of the root canal. *Int Endod J.,* May 2005, vol. 38 (5), 302-9
- **PICK RM.** Lasers in dentistry: where we are today. *Dent Today.,* September 2000, vol. 19 (9), 50-3
- **FERRO D ; BARINOV SM ; RAU JV ; TEGHIL R ; LATINI A.** Calcium phosphate and fluorinated calcium phosphate coatings on titanium deposited by Nd:YAG laser at a high fluence. *Biomaterials,* March 2005, vol. 26 (7), 805-12
- **KOBAYASHI CA ; FUJISHIMA A ; MIYAZAKI T ; KIMURA Y ; MATSUMOTO K ; OSADA T et al.** Effect of Nd: YAG laser irradiation on shear bond strength of glass-Ionomer luting cement to dentin surface. *Int J Prosthodont.,* September 2003, vol. 16 (5), 493-8
- **PIOCH T ; MOLLER D ; STAEHLE HJ ; HOPPE W.** Solubility of enamel and synthetic hydroxyl apatite on irradiation. *Dtsch Zahnarztl Z.,* June 1991, vol. 46 (6), 413-5
- **QUINTANA E ; MÁRQUEZ F ; ROCA I ; TORRES V ; SALGADO J.** Some morphologic changes induced by Nd: YAG laser on the noncoated enamel surface: a scanning electron microscopy study. *Lasers Surg Med.,* 1992, vol. 12 (2), 131-6
- **SWIFT EJ JR ; EDWARDS GS ; PERDIGAO J ; THOMPSON JY ; NUNES MF ; RUDDELL DE et al.** Free-electron laser etching of dental enamel. *J Dent.,* July 2001, vol. 29 (5), 347-53
- **SAZAK H ; TURKMEN C ; GUNDAY M.** Effects of Nd: YAG laser, air-abrasion and acid-etching on human enamel and dentin. *Oper Dent.,* September 2001, vol. 26 (5), 476-81
- **ZENTNER A ; DUSCHNER H.** Structural changes of acid etched enamel examined under confocal laser scanning microscope. *J Orofac Orthop.,* August 1996, vol. 57 (4), 202-9
- **KORYTNICKI D ; MAYER MP ; DARONCH M ; SINGER JDA M ; GRANDE RH.** Effects of Nd:YAG laser on enamel microhardness and dental plaque composition: an in situ study. *Photomed Laser Surg.,* February 2006, vol. 24 (1), 59-63
- **KURAMOTO JUNIOR M ; MATSON E ; TURBINO ML ; MARQUES RA.** Microhardness of Nd:YAG laser irradiated enamel surfaces. *Braz Dent J.,* 2001, vol. 12 (1), 31-3
- **KIELBASSA AM ; WRBAS KT ; SCHULTE-MONTING J ; HELLWIG E.** Correlation of transversal microradiography and microhardness on in situ-induced demineralization in irradiated and nonirradiated human dental enamel. *Aren Oral Biol.,* March 1999, vol. 44 (3), 243-51
- **SALAMA SN ; KINAWI NA.** X-ray study and microhardness data of some dental enamel species. *Biomaterials,* April 1989, vol. 10 (3), 209-12
- **FOSSE G ; ROSENGREN B ; SKAALE S ; LEKNES K ; WULFF L.** An in vivo method for microhardness measurements on human teeth. *Scand J Dent Res.,* 09 February 1986, vol. 4 (1), 27-37
- **DEMETRIADES A ; KOULOURIDES T.** Experimental studies of dental caries. Measurements of enamel microhardness. *Stomatologia,* March 1969, vol. 26 (2), 69-78
- **MÁRQUEZ F ; QUINTANA E ; ROCA I ; SALGADO J.** Physical-mechanical effects of Nd:YAG laser on the surface of sound dental enamel. *Biomaterials,* 1993, vol. 14 (4), 313-6
- **DANILINA TF ; BAGMUTOV VP ; SLAVSKII LUL.** The microhardness of dental tissues as an index of their normal functional stability and in pathological states. *Stomatologiia (Mosk),* 1998, vol. 77 (3), 9-11
- **KLINGER HG ; WIEDEMANN W ; DOCOS E.** Mechanical properties and acid solubility of the dental surface. I. *Microhardness. Dtsch Zahnarztl Z,* August 1980, vol. 35 (8), 823-7
- **FLORIN R ; HERRMANN C ; BERNHARDT W.** Measuring microhardness of laser exposed tooth surface. *Stomatol DDR.,* 04 February 1990, vol. 0 (2), 49-51